# EUROPEAN PATENT APPLICATION

(11) **EP 4 578 299 A1**
(43) Date of publication of application: **02.07.2025**
(21) Application number: 23857406.5
(22) Date of filing: 24.08.2023
(51) Int. Cl.: A23L 33/125, A23L 5/00, A61K 31/702, A61P 1/00, A61P 3/08, A61P 11/00, A61P 25/00, A61P 29/00, A61P 35/00, A23C 9/123, A23C 9/13, A23L 2/02, A23L 2/52

(54) **COMPOSITION FOR IMPROVING INTESTINAL BACTERIAL FLORA**

(30) Priority: 25.08.2022 JP 2022134444
(71) Applicant: MEIJI CO., LTD, Chuo-ku Tokyo 104-8306 (JP)
(72) Inventor: ISHIDA Junya, Hachioji-shi, Tokyo 192-0919 (JP); ICHIKAWA Ayumi, Hachioji-shi, Tokyo 192-0919 (JP); NEGISHI Hiroki, Hachioji-shi, Tokyo 192-0919 (JP)
(74) Representative: TBK
(86) International application number: PCT/JP2023/030455
(87) International publication number: WO 2024/043298

(57) **Abstract**

The object is to increase the relative abundances of *Parabacteroides* bacteria and *Bifidobacterium* bacteria in the intestinal flora. A composition for improvement of intestinal flora is provided, which comprises any selected from the group consisting of kojibiose and oligosaccharides having kojibiose as a constituent sugar.

## Description

### Technical Field

The present invention relates to a composition for improvement of intestinal flora.

### Background Art

In recent years, an intestinal barrier dysfunction called leaky gut, which is caused by lifestyle habits and stress, has become an increasing problem. Inflammation caused by leaky gut is thought to contribute not only to intestinal inflammation but also to the onset and progression of lifestyle-related diseases such as obesity and diabetes, since it spreads toxic substances throughout the body via blood vessels. Improvement of the intestinal flora is being investigated as one of the measures to improve leaky gut.

*Parabacteroides* bacteria constitute one class of the bacteria that inhabit the intestine. With respect to *Parabacteroides* bacteria, for example, Non-patent document 1 examines potential of isolated fractions containing *Hirsutella sinensis* mycelia (HSM) and polysaccharides to modulate the constitution of the intestinal flora, and thereby prevent diet-induced obesity and type II diabetes. This reference reports that a specific fraction selectively promotes the growth of *Parabacteroides goldsteinii,* an indigenous bacterium whose level is reduced in high-fat diet (HFD)-fed mice, and that oral administration of live *P. goldsteinii* to HFD-fed mice reduced obesity, and was associated with increased adipose tissue thermogenesis, improved intestinal integrity, and reduced levels of inflammation and insulin resistance. Non-patent document 2 examines the metabolic effects of *Parabacteroides distasonis* (PD) on weight gain, hyperglycemia, and decreased hepatic steatosis in HFD-fed mice. This reference reports that administration of *P. distasonis* to HFD-fed mice increased lithocholic acid and ursodeoxycholic acid, dramatically altered bile acid profiles, and increased intestinal succinic acid levels. Further, Non-patent document 3 reports that *P*. *distasonis* membrane fraction (PdMb) strongly suppressed the production of inflammatory cytokines in colon cancer cell lines, and decreased the expression amounts of MyD88 and pAkt (ser473) induced by *E. coli* lipopolysaccharides, and PdMb induced apoptosis in colon cancer cell lines and inhibited TLR4 activation in reporter strains. This reference states that these results suggest that *P. distasonis* has anti-inflammatory and anti-cancer effects, possibly via suppression of TLR4 and Akt signaling and promoting apoptosis.

Further, Non-patent document 4 describes that *P. distasonis* is reduced in multiple sclerosis (MS) patients, and Non-patent document 5 reports that multivariate analysis showed that patients with non-alcoholic fatty liver disease (NAFLD) and healthy controls (CTRLs) can be distinguished on the basis of combination of *Oscillospira* bacteria, *Rikenellaceae* bacteria, *Parabacteroides* bacteria and so forth. Non-patent document 6 reports that oral administration of *Parabacteroides goldsteinii* ameliorated weight loss and elevated inflammatory cytokines in lung tissue in a mouse model of smoking-induced chronic obstructive pulmonary disease (COPD). This reference also suggests that the mechanism of such amelioration is that pentaacyl-type lipopolysaccharides, cell wall components of *Parabacteroides goldsteinii,* antagonize the activation of TLR4 receptors by inflammatory hexaacyl-type lipopolysaccharides derived from intestinal bacteria.

Meanwhile, it is known that kojibiose has a growth-promoting effect on bifidobacteria (e.g., Non-patent document 7). With regard to kojibiose and oligosaccharides containing kojibiose as a constituent sugar, Patent document 1 describes an anti-inflammatory agent containing a carbohydrate having an intramolecular ketoaldohexose structure as an active ingredient, and as preferred examples of the carbohydrate having an intramolecular ketoaldohexose structure, mentions 3-ketotrehalose, 3-ketokojibiose, 3-ketoisomaltose, 3-ketolactose, 3-ketomaltose, 3-ketosucrose, and 3-ketomultithol. Further, Patent document 2 describes an in vivo lipid regulator containing one or more selected from kojibiose, kojitriose, kojibiosylglucoside, kojitetraose, and kojitriosylglucoside as an active ingredient.

Furthermore, fructooligosaccharides are used as an involved ingredient in foods for specified health use, and intake thereof is known to increase the growth of bifidobacteria in the large intestine and to produce a bowel regulating effect, either by the growth of bifidobacteria itself or by the action of short-chain fatty acids produced by the bifidobacteria. It is also known that when short-chain fatty acids are produced, the pH in the large intestine decreases, resulting in increased solubility of minerals, which facilitates their passage through mucosal cells and promotes their absorption into the cells (Non-patent document 8).

### Prior Art References

### Patent documents

Patent document 1: WO2017/018336 (Japanese Patent No. 6729913)
Patent document 2: Japanese Patent Publication (Kokai) No. 2005-281188 (Japanese Patent No. 4746281)

### Non-patent documents

Non-patent document 1: Gut commensal Parabacteroides goldsteinii plays a predominant role in the anti-obesity effects of polysaccharides isolated from Hirsutella sinensis. Gut. 2019 Feb;68(2):248-262
Non-patent document 2: Parabacteroides distasonis alleviates obesity and metabolic dysfunctions via production of succinate and secondary bile acids. Cell Rep. 2019 Jan 2;26(1):222-235
Non-patent document 3: Parabacteroides distasonis attenuates toll-like receptor 4 signaling and Akt activation and blocks coon tumor formation in high-fat diet- fed azoxymethane-treated mice. Koh et al. Int. J. Cancer. 2018;143(7):1797-1805 Non-patent document 4: Gut bacteria from multiple sclerosis patients modulate human T cells and exacerbate symptoms in mouse models. Cekanaviciute et al., Proc. Natl. Acad. Sci. USA. 2017;114(70):10713-10718
Non-patent document 5: Gut microbiota profiling of pediatric non-alcoholic fatty liver disease and obese patients unveiled by an integrated meta-omics-based approach. Del Chierico et al., Hepatology. 2017;65(2):451-464
Non-patent document 6: Gut microbiota modulates COPD pathogenesis: role of anti-inflammatory Parabacteroides goldsteinii lipopolysaccharide. Gut microbiota 2022;71, 309-321
Non-patent document 7: Influence of disaccharide structure on prebiotic selectivity in vitro. Journal of agricultural and food chemistry. 2005, 53, 5192-5199
Non-patent document 8: Mineral absorption-promoting effect of non-digestible carbohydrates (fructooligosaccharides). Journal of the Japanese Society of Nutrition and Food Science, Vol. 52, No. 6, 387-395

### Summary of the Invention

### Problem to be solved by the invention

*Parabacteroides* bacteria are considered to constitute one class of the useful intestinal bacteria. However, *Parabacteroides* bacteria are not common probiotic species and have not been normally ingested, so the safety of oral ingestion and passage through gastrointestinal tract of compositions comprising *Parabacteroides* bacteria are unknown. In addition, bifidobacteria are difficult to reach the intestine when they are orally ingested. Increasing the relative abundances of these bacteria in the intestinal flora with specific prebiotics may be a useful means in practical applications.

### Means for solving the problem

The present invention provides the followings.
[1] A composition for improvement of intestinal flora, which comprises any selected from the group consisting of kojibiose and oligosaccharides having kojibiose as a constituent sugar.
[2] The composition according to 1, wherein the improvement of intestinal flora includes promoting growth of *Parabacteroides* bacteria.
[3] A composition for promoting growth of *Bifidobacterium* bacteria in the intestine, which comprises galactosylkojibiose.
[4] The composition according to any one of 1 to 3, which is for use as prebiotics or synbiotics.
[5] A composition for treating any selected from the group consisting of inflammation, chronic obstructive pulmonary disease, obesity, abnormal glucose metabolism, multiple sclerosis, hepatic steatosis, and cancer, which comprises any selected from the group consisting of kojibiose and oligosaccharides having kojibiose as a constituent sugar.
[6] The composition according to 5, wherein the treatment is carried out through promoting growth of *Parabacteroides* bacteria in the intestine.
[7] A composition for treating a disease or condition that is ameliorated by promoting growth of *Parabacteroides* bacteria in the intestines, which comprises any selected from the group consisting of kojibiose and oligosaccharides having kojibiose as a constituent sugar.
[8] A composition for promoting mineral absorption or intestinal regulation, which comprises galactosylkojibiose.
[9] The composition according to 8, wherein the mineral absorption promotion or intestinal regulation is carried out through promoting growth of *Bifidobacterium* bacteria.
[10] A composition for treating a disease or condition that is ameliorated by promoting growth of *Bifidobacterium* bacteria in the intestines, which comprises galactosylkojibiose.
[11] The composition according to any one of 1 to 10, which comprises any selected from the group consisting of kojibiose and oligosaccharides having kojibiose as a constituent sugar as a composition comprising galactosylkojibiose obtained by allowing lactic acid bacteria that express a glucansucrase or a processed product thereof to act on a composition comprising sucrose and lactose.
[12] The composition according to any one of 2, 4, 6, and 7, wherein the *Parabacteroides* bacteria contain *Parabacteroides distasonis.*
[14] A food information-providing apparatus comprising:
   an information acquisition part that acquires information on intestinal flora of a subject;
   a derivation part that derives information on a food to be suggested to the subject on the basis of the information on the intestinal flora; and
   a provision part that provides the derived information on the food to the subject, wherein
   the information acquisition part acquires information on the presence or absence or amount of *Parabacteroides* bacteria from the information on the intestinal flora, and the derivation part derives information on the food that is a composition comprising any selected from the group consisting of kojibiose and oligosaccharides having kojibiose as a constituent sugar on the basis of the information on the presence or absence or amount of *Parabacteroides* bacteria, or
   the information acquisition part acquires information on the presence or absence or amount of *Bifidobacterium* bacteria from the information on the intestinal flora, and the derivation part derives information on the food that is a composition comprising galactosylkojibiose on the basis of the information on the presence or absence or amount of *Bifidobacterium* bacteria.
[15] The apparatus according to 14, wherein the information on the food to be provided can be displayed on a terminal of the subject.
[16] The apparatus according to 14 or 15, which further comprises a display part that displays the information on the food to be provided.
[17] A method for providing information on a food, which comprises the steps of
   acquiring information on intestinal flora of a subject;
   deriving information on a food to be suggested to the subject on the basis of the information on the intestinal flora; and
   providing the derived information on the food to the subject,
   wherein
   in the step of acquiring the information, information on the presence or absence or amount of *Parabacteroides* bacteria is acquired from the information on the intestinal flora, and in the step of deriving the information on the food, information on the food that is a composition comprising any selected from the group consisting of kojibiose and oligosaccharides having kojibiose as a constituent sugar is derived on the basis of the information on the presence or absence or amount of *Parabacteroides* bacteria, or
   in the step of acquiring the information, information on the presence or absence or amount of *Bifidobacterium* bacteria is acquired from the information on the intestinal flora, and in the step of deriving the information on the food, information on the food that is a composition comprising galactosylkojibiose is derived on the basis of the information on the presence or absence or amount of *Bifidobacterium* bacteria.
[18] The method according to 17, which further comprises the step of displaying the information on the food to be provided on a terminal of the subject.
[19] The apparatus according to any one of 14 to 16, or the method according to 17 or 18, wherein the *Parabacteroides* bacteria consist of *Parabacteroides distasonis.*
[21] A composition for use in a method for improvement of intestinal flora, the composition comprising any selected from the group consisting of kojibiose and oligosaccharides having kojibiose as a constituent sugar; use of any selected from the group consisting of kojibiose and oligosaccharides having kojibiose as a constituent sugar in manufacturing a composition for improvement of intestinal flora; a method for improvement of intestinal flora, which comprises the step of administering a composition comprising any selected from the group consisting of kojibiose and oligosaccharides having kojibiose as a constituent sugar to a subject; or use of a composition comprising any selected from the group consisting of kojibiose and oligosaccharides having kojibiose as a constituent sugar, which is for improvement of intestinal flora.
[22] The composition, use in the production, method, or use according to 21, wherein the improvement of the intestinal flora includes promoting growth of *Parabacteroides* bacteria.
[23] A composition comprising galactosylkojibiose, which is for use in a method for promoting growth of *Bifidobacterium* bacteria in the intestine; use of a composition comprising galactosylkojibiose in manufacturing a composition for promoting growth of *Bifidobacterium* bacteria in the intestine; a method for promoting growth of *Bifidobacterium* bacteria in the intestine, which comprises the step of administering a composition comprising galactosylkojibiose to a subject; or use of a composition comprising galactosylkojibiose, which is for promoting growth of *Bifidobacterium* bacteria in the intestine.
[24] The composition according to any one of 21 to 23, which is for use as prebiotics or synbiotics; use of such a composition in manufacturing according to any one of 21 to 23; the method according to any one of 21 to 23, which comprises the step of administering the composition as prebiotics or synbiotics; or use of the composition according to any one of 21 to 23 as prebiotics or synbiotics.
[25] A composition comprising any selected from the group consisting of kojibiose and oligosaccharides having kojibiose as a constituent sugar, which is for use in a method for treating any selected from the group consisting of inflammation, chronic obstructive pulmonary disease, obesity, abnormal glucose metabolism, multiple sclerosis, hepatic steatosis, and cancer; use of any selected from the group consisting of kojibiose and oligosaccharides having kojibiose as a constituent sugar in manufacturing a composition for treating any selected from the group consisting of inflammation, chronic obstructive pulmonary disease, obesity, abnormal glucose metabolism, multiple sclerosis, hepatic steatosis, and cancer; a method for treating any selected from the group consisting of inflammation, chronic obstructive pulmonary disease, obesity, abnormal glucose metabolism, multiple sclerosis, hepatic steatosis, and cancer, which comprises the step of administering a composition comprising any selected from the group consisting of kojibiose and oligosaccharides having kojibiose as a constituent sugar to a subject; or use of a composition comprising any one selected from the group consisting of kojibiose and oligosaccharides having kojibiose as a constituent sugar for treating any selected from the group consisting of inflammation, chronic obstructive pulmonary disease, obesity, abnormal glucose metabolism, multiple sclerosis, hepatic steatosis, and cancer.
[26] The composition, use in manufacturing, method, or use according to 25, wherein the treatment is carried out through promoting growth of *Parabacteroides* bacteria in the intestine.
[27] A composition comprising any selected from the group consisting of kojibiose and oligosaccharides having kojibiose as a constituent sugar, which is for use in a method for treating a disease or condition that is ameliorated by promoting growth of *Parabacteroides* bacteria in the intestines; use of any selected from the group consisting of kojibiose and oligosaccharides having kojibiose as a constituent sugar in manufacturing a composition for treating a disease or condition that is ameliorated by promoting growth of *Parabacteroides* bacteria in the intestine; a method for treating a disease or condition that is ameliorated by promoting growth of *Parabacteroides* bacteria in the intestine, which comprises the step of administering a composition comprising any selected from the group consisting of kojibiose and oligosaccharides having kojibiose as a constituent sugar to a subject; or use of a composition comprising any selected from the group consisting of kojibiose and oligosaccharides comprising kojibiose as a constituent sugar for treating a disease or condition that is ameliorated by promoting growth of *Parabacteroides* bacteria in the intestine.
[28] A composition comprising any selected from the group consisting of kojibiose and oligosaccharides having kojibiose as a constituent sugar, which is for promoting mineral absorption or intestinal regulation; use of any selected from the group consisting of kojibiose and oligosaccharides having kojibiose as a constituent sugar in manufacturing a composition for promoting mineral absorption or intestinal regulation; a method for promoting mineral absorption or intestinal regulation, which comprises the step of administering a composition comprising any selected from the group consisting of kojibiose and oligosaccharides having kojibiose as a constituent sugar to a subject; or use of a composition comprising any selected from the group consisting of kojibiose and oligosaccharides having kojibiose as a constituent sugar, which is for promoting mineral absorption or intestinal regulation.
[29] The composition, use in manufacturing, method, or use according to 28, wherein the mineral absorption promotion or intestinal regulation is carried out through promoting promoting growth of *Bifidobacterium* bacteria in the intestines.
[30] A composition comprising galactosylkojibiose, which is for use in a method for treating a disease or condition that is ameliorated by promoting growth of *Bifidobacterium* bacteria in the intestines; use of a composition comprising galactosylkojibiose in manufacturing a composition for treating a disease or condition that is ameliorated by promoting growth of *Bifidobacterium* bacteria in the intestine; a method for treating a disease or condition that is ameliorated by promoting growth of *Bifidobacterium* bacteria in the intestine, which comprises the step of administering a composition comprising galactosylkojibiose to a subject; or use of a composition comprising galactosylkojibiose, which is for promoting growth of *Bifidobacterium* bacteria in the intestine.
[31] The composition, use in manufacturing, method, or use according to any one of 21 to 30, wherein the composition contains any selected from the group consisting of kojibiose and oligosaccharides having kojibiose as a constituent sugar as a composition comprising galactosylkojibiose obtained by allowing lactic acid bacteria that express a glucansucrase or a processed product thereof to act on a composition comprising sucrose and lactose.
[32] The composition according to any one of 22, 24, 26, and 27, wherein the *Parabacteroides* bacteria contain *Parabacteroides distasonis.*

### Effects of the Invention

According to the present invention, intestinal bacterial flora can be improved. In particular, increase of *Parabacteroides* bacteria and *Bifidobacterium* bacteria in the intestinal flora can be promoted.

It can be expected that, through promoting growth of *Parabacteroides* bacteria in the intestinal flora, any selected from the group consisting of inflammation, chronic obstructive pulmonary disease, obesity, abnormal glucose metabolism, multiple sclerosis, hepatic steatosis, and cancer can be treated, and various diseases or conditions can be treated by promoting growth of *Parabacteroides* bacteria and *Bifidobacterium* bacteria in the intestinal flora.

Functional foods in various forms can be provided by adding the active ingredient of the present invention.

### Brief Description of the Drawings

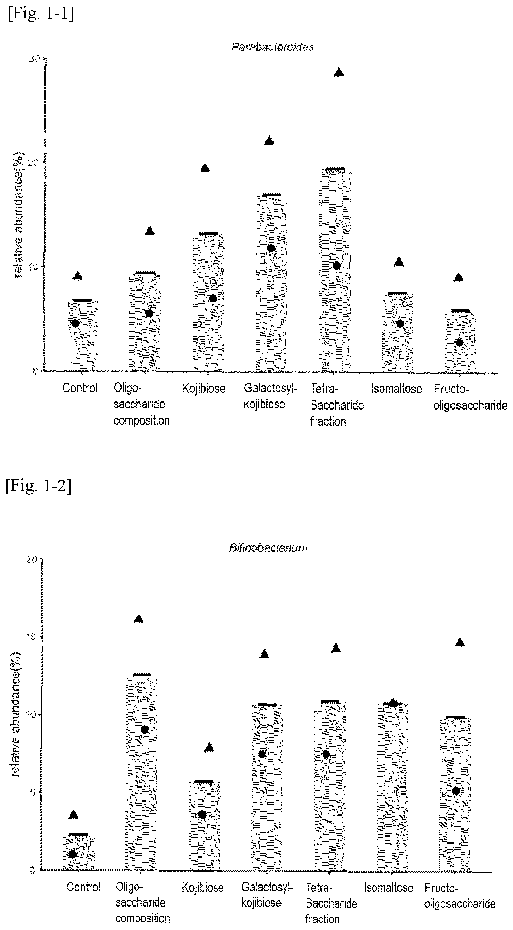

[Fig. 1-1] Changes in the relative abundance of *Parabacteroides* bacteria induced by various carbohydrates in a human fecal culture system. Black circles and black triangles indicate the results for the subjects respectively, and horizontal bars indicate mean values.
[Fig. 1-2] Changes in the relative abundance of *Bifidobacterium* bacteria induced by various carbohydrates in a human fecal culture system. Black circles and black triangles indicate the results for the subjects, and horizontal bars indicate mean values.
[Fig. 2] Changes in the relative abundances of *Parabacteroides* and *Bifidobacterium* bacteria in the intestinal flora observed after ingestion of 20 g per day of a sugar solution containing kojibiose and galactosylkojibiose for 2 weeks. Black circles indicate the results for the subjects.
[Fig. 3-1] Changes in the relative abundance of *Parabacteroides* bacteria induced by galactosylkojibiose (trisaccharide fraction) in a human fecal culture system determined on the basis of the amplicon sequencing of the V3-V4 region performed with MiSeq (Illumina). Black circles indicate the results for the subjects, and horizontal bars indicate mean values.
[Fig. 3-2] Changes in the relative abundance of species identified as *Parabacteroides distasonis* induced by galactosylkojibiose (trisaccharide fraction) in a human fecal culture system determined by species-level analysis performed by amplicon sequencing of the V3-V4 region using MiSeq (Illumina). Black circles indicate the results for the subjects and horizontal bars indicate mean values.
[Fig. 4] A chromatogram of an enzymatic reaction solution obtained by allowing glucansucrase to act on a mixture of sucrose and lactose.
[Fig. 5] Chromatograms of the enzymatic reaction solution and the fractionated and purified trisaccharide and tetrasaccharide fractions.
[Fig. 6] Generation of kojibiose by a lactase treatment of the trisaccharide fraction.

### Modes for Carrying out the Invention

The present invention relates to a composition for promoting growth of *Parabacteroides* bacteria (bacteria belonging to the genus *Parabacteroides*) in intestinal flora and for promoting growth of *Bifidobacterium* bacteria (bacteria belonging to the genus *Bifidobacterium*) in the intestinal flora, which comprises kojibiose or an oligosaccharide having kojibiose as a constituent sugar as an active ingredient.

### [Active ingredient]

The composition of the present invention contains any selected from the group consisting of kojibiose and oligosaccharides having kojibiose as a constituent sugar as an active ingredient. The term "oligosaccharide having kojibiose as a constituent sugar" does not include kojibiose itself. For the present invention, the term "any" is used in the sense of "at least one" unless especially stated. For example, "any selected from the group consisting of kojibiose and oligosaccharides having kojibiose as a constituent sugar" means only kojibiose or only one type of oligosaccharide having kojibiose as a constituent sugar, as well as kojibiose and one type of oligosaccharide having kojibiose as a constituent sugar, two types of oligosaccharides having kojibiose as a constituent sugar, and so forth.

The kojibiose and oligosaccharides having kojibiose as a constituent sugar used in the composition are not particularly limited as long as they have the desired effect. The kojibiose and oligosaccharides having kojibiose as a constituent sugar used in the composition may consist of one type of saccharide or a combination of two or more types of saccharides.

Examples of the oligosaccharides having kojibiose as a constituent sugar include galactosylkojibiose, kojitriose, selaginose (kojibiosylglucoside), centose, kojibiosylfructoside, which are trisaccharides, kojitetraose and kojitriosylglucoside, which are tetrasaccharides consisting of any of the foregoing trisaccharides and one molecule of monosaccharide such as glucose attached to the trisaccharide.

In a preferred embodiment, the composition contains any of kojibiose and galactosylkojibiose. Examples of such a composition include a composition comprising kojibiose, composition comprising galactosylkojibiose, composition comprising kojibiose and galactosylkojibiose, composition comprising kojibiose, galactosylkojibiose, and tetrasaccharide consisting of galactosylkojibiose and one molecule of glucose attached to the galactosylkojibiose.

For the present invention, galactosylkojibiose refers to kojibiose mentioned below having galactose linked at any of the positions 1 to 8 of the kojibiose through α-or β-linkage, unless especially noted. Theoretically, galactose can link to the OH at the position 8 in four ways; α-α, α-β, β-α, and β-β, and to the OH at the positions 2 to 7 in two ways, and therefore 18 types of galactosylkojibioses can be assumed. They are referred to as 8α-α kojibiose, 8α-β kojibiose, 8β-α kojibiose, 8β-β kojibiose, 1α kojibiose, 1β kojibiose, 2α kojibiose, 2β kojibiose, 3α kojibiose, 3β kojibiose, 4α kojibiose, 4β kojibiose, 5α kojibiose, 5β kojibiose, 6α kojibiose, 6β kojibiose, 7α kojibiose, and 7β kojibiose, respectively.

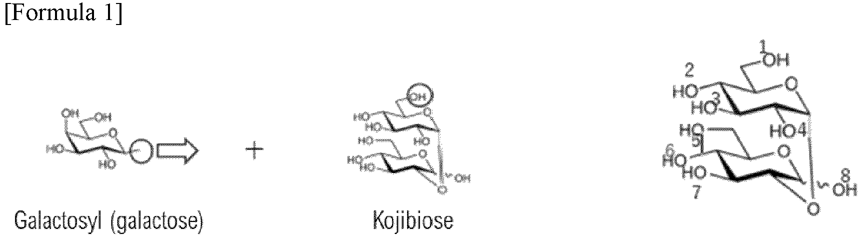

In one embodiment, the composition contain any one of the 18 types of galactosylkojibioses, namely, 8α-α kojibiose, 8α-β kojibiose, 8β-α kojibiose, 8β-β kojibiose, 1α kojibiose, 1β kojibiose, 2α kojibiose, 2β kojibiose, 3α kojibiose, 3β-kojibiose, 4α-kojibiose, 4β-kojibiose, 5α-kojibiose, 5β-kojibiose, 6α-kojibiose, 6β-kojibiose, 7α-kojibiose, and 7β-kojibiose, or may contain 2, 3, 4, 5, 6, 7, 8, 9, 10, 11, 12, 13, 14, 15, 16, 17, or 18 types of galactosylkojibioses.

In a preferred embodiment, the galactosylkojibiose contained in the composition is α-D-glucopyranosyl-(1→2)-[β-D-galactopyranosyl-(1→4)-]D-glucopyranoside). This sugar can also be described as one type of glucosyllactose.

According to the studies of the inventors of the present invention, kojibiose and galactosylkojibiose have been confirmed to have excellent growth-promoting effects on *Parabacteroides* and *Bifidobacterium* bacteria. The oligosaccharides having kojibiose as a constituent sugar herein referred to can produce kojibiose in the digestive tract of a subject after administration to the subject. For example, galactosylkojibiose is degraded by lactase to generate kojibiose. Therefore, the desired effect can be obtained with any saccharide having kojibiose as a constituent sugar.

Origin and production method of the kojibiose and oligosaccharides having kojibiose as a constituent sugar used in the present invention are not particularly limited, and they may be those produced by any of fermentation method, enzymatic method, organic synthesis method, and so forth. One of the preferred production methods is a production method comprising the step of allowing a glucansucrase or lactic acid bacteria that express a glucansucrase or a processed product thereof to act on a composition comprising lactose and sucrose to obtain a composition comprising galactosylkojibiose. For the present invention, the glucansucrase is an enzyme belonging to glycoside hydrolase (also called glycosyl hydrolase) family 70 (GH70), unless especially stated (see (CAZy) databases, and Cantarel et al., Nucleic Acids Res. 37:D233-238, 2009).

When lactic acid bacteria are used, it is preferred that they are lactic acid bacteria that express a glucansucrase. Examples of preferred lactic acid bacteria are lactic acid bacteria belonging to the family *Leuconostoc,* more preferably lactic acid bacteria belonging to the genus *Leuconostoc,* further preferably *Leuconostoc mesenteroides.*

When lactic acid bacteria are used, it is preferred that they are lactic acid bacteria that constitutively express a glucansucrase. Examples of preferred lactic acid bacteria are lactic acid bacteria belonging to the family *Lactobacillaceae,* more preferably lactic acid bacteria belonging to the genus *Liquorilactobacillus* or *Limosilactobacillus,* further preferably *Limosilactobacillus reuteri* or *Liquorilactobacillus satsumensis.*

When lactic acid bacteria are used, it is preferred that they are lactic acid bacteria that constitutively express a glucansucrase and the glucansucrase is more preferably of bacterial cell-bound type. Examples of preferred lactic acid bacteria are lactic acid bacteria belonging to the family *Lactobacillaceae,* more preferably lactic acid bacteria belonging to the genus *Liquorilactobacillus,* further preferably *Liquorilactobacillus satsumensis.* An example of particularly preferred strains of *Liquorilactobacillus satsumensis* is JCM12392. JCM12392 is described as *Lactobacillus satsumensis* in RIKEN BioResource Center, GENERAL CATALOG No. 9, 2012, JAPAN COLLECTION OF MICROORGANISMS, M51.

When lactic acid bacteria are explained in connection with the present invention, they are explained according to the taxonomy including the reclassification by Zheng J, Wittouck S, Salvetti E, Franz CMAP, Harris HMB, Mattarelli P, O'Toole PW, Pot B, Vandamme P, Walter J, Watanabe K, Wuyts S, Felis GE, Ganzle MG, Lebeer S.: A taxonomic note on the genus Lactobacillus: Description of 23 novel genera, emended description of the genus Lactobacillus Beijerinck 1901, and union of Lactobacillaceae and Leuconostocaceae. Int J Syst Evol Microbiol. 2020 Apr; 70(4): 2782-2858, unless especially indicated. According to the taxonomy used before the reclassification, *Liquorilactobacillus satsumensis* was classified as *Lactobacillus satsumensis.*

The lactic acid bacteria that express a glucansucrase or a processed product thereof is preferably any selected from the group consisting of live bacterial cells, dead bacterial cells, cultures containing bacterial cells, disrupted bacterial cells, and a purified product of a glucansucrase.

The glucansucrase or lactic acid bacteria that express a glucansucrase or a processed product thereof used for the production of kojibiose or an oligosaccharide having kojibiose as a constituent sugar can be produced by using transformation, chemical synthesis, or genome edition techniques.

The composition comprising galactosylkojibiose obtained by such a production method can be used as a solution containing kojibiose or an oligosaccharide having kojibiose as a constituent sugar as it is, or used after purification with an ion exchange resin or the like. That is, the composition of the present invention may be a composition comprising galactosylkojibiose, which can be obtained by allowing a glucansucrase or lactic acid bacteria that express a glucansucrase or a processed product thereof to act on a composition comprising sucrose and lactose.

### [Use]

The composition of the invention can be used for promoting growth of *Parabacteroides* bacteria in the intestinal flora and for promoting growth of *Bifidobacterium* bacteria in the intestinal flora.

For the present invention, the *Parabacteroides* bacteria are bacteria identified as belonging to the genus *Parabacteroides* by molecular phylogenetic analysis based on the 16S rRNA gene, and the same applies to the *Bifidobacterium* bacteria. The criteria for determining the genus by molecular phylogenetic analysis are well known to those skilled in the art (Stackebrandt E, Ebers J. Taxonomic parameters revisited: tarnished gold standards. Microbiol Today 2006;33:152-155.). Examples of the *Parabacteroides* bacteria include *Parabacteroides distasonis, Parabacteroides merdae, Parabacteroides acidifaciens, Parabacteroides faecis, Parabacteroides goldsteinii,* and *Parabacteroides johnsonii.* Examples of the *Bifidobacterium* bacteria include *Bifidobacterium breve, Bifidobacterium longum* subsp. *infantis, Bifidobacterium bifidum, Bifidobacterium longum* subsp. *longum, Bifidobacterium adolescentis, Bifidobacterium catenulatum, Bifidobacterium pseudocatenulatum, Bifidobacterium angulatum,* and *Bifidobacterium dentium.*

In a preferred embodiment, the composition is for promoting growth of any selected from *Parabacteroides merdae, Parabacteroides johnsonii,* and *Parabacteroides goldsteinii,* which are abundantly present in the intestine, among *Parabacteroides* bacteria.

For the present invention, promoting growth of specific bacteria in the flora means increasing proportion (relative abundance) of the specific bacteria in the bacterial flora.

Whether or not a certain ingredient promotes growth of specific bacteria in the intestinal flora can be evaluated as follows. Feces provided by a healthy person is added to an appropriate medium, and incubated for a certain period of time as required, then an ingredient desired to be evaluated is added, the mixture is incubated under appropriate conditions (e.g., 37°C for 48 hours under an anaerobic condition similar to those in the intestine), and the relative abundance of the specific bacteria in the bacterial flora in the culture is measured. The result of the measurement can then be compared with the result of a culture incubated under otherwise identical conditions, differing only in that a control (e.g., sterile water) was added instead of the ingredient desired to be evaluated, and if the relative abundance is higher than that provided by the control, it can be judged that growth is promoted, and if lower, growth is inhibited.

The relative abundance of particular bacteria in the bacterial flora can be determined by known methods. One preferred method is to perform 16S metagenomic analysis (sequence analysis of 16S rRNA gene amplicons) on DNA extracted from cultures. When the 16S metagenomic analysis is performed, DNA can be extracted by using commercially available kits. The genomic region to be analyzed is not particularly limited as long as the bacteria can be determined, but the V3-V4 region of the 16S rRNA gene can be used. As primers, amplification conditions, purification method of amplicons, and so forth for analyzing bacteria, materials and methods well known to those skilled in the art can be used. Sequence decoding is preferably performed on a next-generation sequencer with higher performance. Next-generation microbiome bioinformatics platforms such as QIIME 2^{™} can be used to analyze the obtained data. For the 16S metagenomic analysis, those skilled in the art can refer to information such as Sanschagrin S, Yergeau E. Next-generation sequencing of 16S ribosomal RNA gene amplicons. J Vis Exp. 2014;(90):51709. Published 2014 Aug 29. doi:10.3791/51709, etc.

The *Parabacteroides* bacteria referred to in the present invention are bacteria that inhabit in the intestines. Promotion of growth of *Parabacteroides* bacteria is expected to have effects against inflammation, obesity, abnormal glucose metabolism, hepatic steatosis, and cancer (Non-patent documents 1 to 3). In addition, it has been reported that *P. distasonis* is decreased in multiple sclerosis (MS) patients (Non-patent document 4), and patients with non-alcoholic fatty liver disease (NAFLD) and healthy controls (CTRLs) can be distinguished on the basis of the combination of *Parabacteroides* bacteria with *Oscillospira, Rickenellaceae, Parabacteroides* bacteria, etc. (Non-patent document 5). Therefore, the composition can be used for treating any selected from the group consisting of inflammation, obesity, abnormal glucose metabolism, multiple sclerosis (MS), hepatic steatosis, and cancer.

It has also been reported that oral administration of *Parabacteroides goldsteinii* ameliorated weight loss and elevated inflammatory cytokines in lung tissue in a mouse model of smoking-induced chronic obstructive pulmonary disease (COPD), and it has been suggested that pentaacyl-type lipopolysaccharides, cell wall components of *Parabacteroides goldsteinii,* antagonize the activation of TLR4 receptors by inflammatory hexaacyl-type lipopolysaccharides derived from intestinal bacteria as the mechanism of the above amelioration (Non-patent document 6). Therefore, the composition can be used for treating chronic obstructive pulmonary disease (COPD).

Furthermore, it is known that bifidobacteria themselves or the short-chain fatty acids produced by bifidobacteria produce a intestine regulating effect, and that the production of short-chain fatty acids lowers the pH in the colon, resulting in increased solubility of minerals, facilitating their passage through mucosal cells, and promoting absorption thereof into the cells (Non-patent document 8). Therefore, the composition can be used for intestinal regulation and to promote absorption of minerals. Examples of such minerals include calcium, magnesium, iron, potassium, zinc, copper, chromium, manganese, molybdenum, iodine, sodium, phosphorus, and selenium. The composition can be expected to promote the absorption of, in particular, calcium, magnesium, and iron, among these.

Inflammation includes inflammatory bowel diseases. Inflammatory bowel diseases include ulcerative colitis and Crohn's disease. Obesity is defined as excessive accumulation of fat in the body (BMI of 25 or higher). If adverse health effects are exhibited due to obesity or visceral adiposity is exhibited, it is called obesitic disease. Abnormal glucose metabolism means a state that abnormality is observed in glucose metabolism, including insulin resistance and diabetes mellitus. Insulin resistance refers to a state in which insulin is secreted into the blood by the pancreas, but the sensitivity of a target organ to insulin is decreased and its action is blunted. In the state of insulin resistance, the glucose uptake capacity of muscle and adipose tissue is reduced and gluconeogenesis is not suppressed in the liver, resulting in difficulty in lowering blood glucose levels and requiring more insulin to return blood glucose levels to normal. It is said that if this condition persists, insulin secretion function of the pancreas declines and blood glucose level rises, which causes type II diabetes. Insulin resistance includes those caused by heredity, obesity, lack of exercise, high-fat diet, and stress. Diabetes mellitus is a disease with chronic high blood sugar due to inadequate insulin action and includes both type I and type II diabetes mellitus. Multiple sclerosis (MS) is a multiple demyelinating disease of the central nervous system (brain, spinal cord, and optic nerves) with multiple spatial and temporal manifestations, including those of remitting-relapsing type, primary progressive type, and secondary progressive type. Hepatic steatosis includes fatty liver (fatty liver disease), alcoholic fatty liver, non-alcoholic fatty liver disease (NAFLD), and simple fatty liver. Cancer includes lung cancer, breast cancer, stomach cancer, colorectal cancer, liver cancer, kidney cancer, pancreatic cancer, uterine cancer, ovarian cancer, head and neck cancer, osteosarcoma, chondrosarcoma, rhabdomyosarcoma, leiomyosarcoma, fibrosarcoma, liposarcoma, hemangiosarcoma, leukemia, malignant lymphoma, and myeloma. Chronic obstructive pulmonary disease (COPD) is sometimes referred to as emphysema or chronic bronchitis. The main cause of COPD is smoking, and it is said that onset of COPD affects not only the lung but also all organs in the body, making people more susceptible to diabetes, arteriosclerosis, osteoporosis, peptic ulcers, and other diseases.

The composition can also be used for treating diseases or conditions that are ameliorated by promoting growth of *Parabacteroides* bacteria in the intestine. Examples of such diseases or conditions may include inflammation, chronic obstructive pulmonary disease (COPD), obesity, abnormal glucose metabolism, multiple sclerosis (MS), hepatic steatosis, and cancer, as well as many others.

The composition can also be used for treating diseases or conditions that are ameliorated by promoting growth of *Bifidobacterium* bacteria in the intestine. Examples of the treatment of such diseases or conditions may include intestinal regulation, promotion of mineral absorption, and many others.

For the present invention, the term treatment referred to for a disease or condition includes reducing the risk of developing the disease or condition, delaying or preventing the development of the disease or condition, treating the disease or condition, and stopping or delaying the progression of the disease or condition. Actions for amelioration or treatment include medical actions performed by physicians and nurses, midwives, and others under the direction of physicians, and non-therapeutic actions performed by non-physicians, such as pharmacists, nutritionists (including registered dietitian nutritionists and sports nutritionists), public health nurses, midwives, nurses, clinical laboratory technicians, sports instructors, drug manufacturers, drug sellers, food manufacturers, food sellers, and others. Furthermore, prevention or reduction of the risk of developing disease or condition includes recommendations for intake of specific foods and nutritional guidance (including guidance on nutrition necessary for medical treatment of injured or sick persons and guidance on nutrition for the maintenance and promotion of good health).

### [Composition]

### (Food compositions, etc.)

The composition of the present invention can be in the form of a food composition or pharmaceutical composition. Food and pharmaceutical are not limited to those for humans, and may be those for animals other than human, unless especially indicated. The food may be a common food, functional food, or nutritional composition, or a therapeutic diet (diet for the purpose of therapy, which is prepared in accordance with a menu prepared by a dietitian or the like according to a dietary prescription issued by a physician), dietetic food, ingredient-modified food, care food, or therapy-supporting food, unless especially indicated. The food is not limited to a solid food, but it may be a food in the form of liquid, for example, drink, drinkable preparation, liquid food, or soup, unless especially indicated. Functional food refers to a food that can give a predetermined functionality to a living body, and includes health foods at large, such as foods for specified health uses (abbreviated as "Tokuho" in Japanese, including conditional foods for specified health use), foods with function claims, foods with health claims including foods with nutrient function claims, foods for special dietary uses, nutritional supplement foods, health supplement foods, supplements (for example, those of various kinds of dosage forms such as tablet, coated tablet, sugar-coated tablet, capsule and solution), and cosmetic food (for example, diet foods). In the present invention, the "functional foods" include health foods to which the health claim based on the food standards of CODEX (JOINT FAO/WHO FOOD STANDARDS PROGRAMME CODEX ALIMENTARIUS COMMISSION) is applied. The composition may also be a purified product of a particular sugar. Such a purified product includes fractionated, partially purified, and crude purified products.

### (Subject)

The composition of the invention is suitably administered to a subject for whom it is desirable or necessary to promote growth of *Parabacteroides* bacteria in the intestine; a subject for whom it is desirable or necessary to promote growth of *Bifidobacterium* bacteria in the intestine; a subject with any selected from the group consisting of inflammation, chronic obstructive pulmonary disease (COPD), obesity, abnormal glucose metabolism, multiple sclerosis (MS), hepatic steatosis, and cancer; a smoker; a subject selected from the group consisting of a subject with constipation or diarrhea or who is prone to mineral deficiency, and a subject for whom it is desirable or necessary to supplement minerals; a subject with a disease or condition that is ameliorated by promoting growth of *Parabacteroides* bacteria in the intestine; and a subject with a disease or condition that is ameliorated by promoting growth of *Bifidobacterium* bacteria in the intestine. The subject for whom it is desirable or necessary to promote growth of *Parabacteroides* bacteria in the intestine includes those with low numbers of *Parabacteroides* bacteria in the intestine. The subject for whom it is desirable or necessary to promote growth of *Bifidobacterium* bacteria in the intestine includes those with low numbers of *Bifidobacterium* bacteria in the intestine. For the present invention, the term "administration" is used in the sense of administering a pharmaceutical product to a subject as well as having a subject ingest a food other than a pharmaceutical product.

The age of the subject is not particularly limited, and the subject may be, for example, a neonate (within 28 days of birth); an infant (younger than 1 year of age); a young child (1 to 6 years of age); a child (7 years of age or older and younger than 15 years of age); an adult (15 years of age or older); or a person of 65 years of age or older.

### (Administration route etc.)

The composition of the present invention may be administered orally, parenterally, e.g., via a tube (gastrostomy or enterostomy), or intranasally, but oral administration is preferred.

The composition can be administered to a subject repeatedly or continuously over a long period of time. The period of time is not particularly limited, but in order for the effect to be fully recognized, the composition should be preferably administered continuously over a relatively long period of time, e.g., over 3 days or longer, 1 week or longer, 2 weeks or longer, 1 month or longer, 3 months or longer, 6 months or longer, or 1 year or longer.

The composition may be administered routinely, in advance, such as when the risk is high, or when the need arises. The composition may be administered as a meal, before, after, or between meals, or when the disease or condition that is desired to be ameliorated by the composition occurs.

### (Dose and content)

The dose of the composition of the present invention may be such an amount that the desired effect is exhibited. The dose can be appropriately set in consideration of various factors such as the age, weight, and symptoms of the subject.

The daily dose of the composition can be 0.5 g or more, or 1 g or more, and is preferably 2 g or more, preferably 3 g or more, more preferably 5 g or more, further preferably 10 g or more, in terms of the amount of the active ingredient. The maximum amount of the active ingredient per day can be 80 g or less, 70 g or less, 60 g or less, 50 g or less, or 40 g or less, or may be 30 g or less, 20 g or less, or 15 g or less, no matter how the minimum amount is defined. When two or more types of active ingredients are contained in the composition, the amount of active ingredient means the total amount of the active ingredients contained.

Administration may be once a day or multiple times a day, e.g., 2 to 10 times. The dose of the active ingredient per one time can be, for example, 0.5 g or more, or 1 g or more, and is preferably 2 g or more,, more preferably 3 g or more, further preferably 5 g or more. The maximum amount of the active ingredient per one time can be 70 g or less, 60 g or less, 50 g or less, or 40 g or less, or may be 30 g or less, 25 g or less, or 10 g or less, no matter how is the minimum amount is defined.

The content of the active ingredient in the composition may be appropriately determined according to the form of the composition. For example, when the composition is in a form to be consumed or drunk as it is, such as fermented milk or beverage, the content of the active ingredient based on weight of the composition can be 0.01% or more, and is preferably 0.1% or more, more preferably 0.3% or more, further preferably 0.5% or more. The maximum amount of the active ingredient based on weight of the composition can be 8% or less, 5% or less, or 4% or less, or may be 3% or less, no matter how the minimum amount is defined. Alternatively, the content of the active ingredient based on solid content of the composition can be 0.1% or more, and is preferably 1% or more, more preferably 3% or more, further preferably 5% or more. The maximum amount of the active ingredient based on the solid content can be 80% or less, 50% or less, or 40 % or less, or may be 30 % or less, no matter how the minimum amount is defined. For the present invention, % means mass percent unless especially stated.

### (Other ingredients and additives)

The composition of the present invention may contain other active ingredients or nutritional ingredients that are acceptable for foods or pharmaceuticals. Examples of such ingredients include lipids (e.g., milk fat, vegetable fats, and fats containing medium-chain fatty acids), proteins (e.g., milk proteins, milk protein concentrate (MPC), whey protein concentrate (WPC), whey protein isolate (WPI), α-lactalbumin (α-La), β-lactoglobulin (β-Lg), heat-denatured whey proteins, and enzyme-treated whey proteins, amino acids (e.g., lysine, arginine, glycine, alanine, glutamic acid, leucine, isoleucine, and valine), carbohydrates other than kojibiose and oligosaccharides having kojibiose as a constituent sugar (e.g., glucose, sucrose, fructose, maltose, trehalose, erythritol, maltitol, palatinose, xylitol, and dextrin), electrolytes (e.g., sodium, potassium, calcium, and magnesium salts), vitamins (e.g., vitamin A, vitamin B1, vitamin B2, vitamin B6, vitamin B12, vitamin C, vitamin D, vitamin E, vitamin K, biotin, folic acid, pantothenic acid, and nicotinic acids), minerals (e.g., copper, zinc, iron, cobalt, and manganese), antibiotics, dietary fibers, and so forth.

For the present invention, the composition may contain prebiotics other than the active ingredient. Prebiotics can be defined as non-digestible food ingredients that selectively alter the growth and activity of certain bacteria in the colon, thereby favorably affecting the host and improving host health. One or more types of prebiotics other than the active ingredient may be used in the composition.

Such prebiotics other than the active ingredient are not particularly limited as long as they do not interfere with the effect of the active ingredient contained in the composition. Examples of the prebiotics other than the active ingredient include galactooligosaccharides, fructooligosaccharides (GF2, GF3, and GF4), xylooligosaccharides, isomaltooligosaccharides, raffinose, lactulose, lactosucrose, soy oligosaccharides, coffee oligosaccharides, dietary fibers and gluconic acid.

The composition may also be used as synbiotics and may contain probiotics in addition to the active ingredient. Synbiotics are a combination of probiotics and prebiotics. Probiotics can be defined as microorganisms that, when introduced into the intestine of a host in a live state, produce beneficial effects for the host.

Probiotics are not particularly limited as long as they do not interfere with the effects of the active ingredient contained in the composition. Examples of probiotics are known to include certain lactic acid bacteria.

The composition may further contain additives that are acceptable for foods or pharmaceuticals. Examples of such additives include inert carriers (solid or liquid carriers), excipients, surfactants, binders, disintegrants, lubricants, dissolution aids, suspending agents, coating agents, colorants, preservatives, buffers, pH adjusters, emulsifiers, stabilizers, sweeteners, antioxidants, flavorings, acidifiers, and natural products. More specifically, examples include water, other aqueous solvents, pharmaceutically acceptable organic solvents, collagen, polyvinyl alcohol, polyvinyl pyrrolidone, carboxyvinyl polymer, sodium alginate, water-soluble dextran, water-soluble dextrin, sodium carboxymethyl starch, pectin, xanthan gum, gum arabic, casein, gelatin, agar, glycerin, propylene glycol, polyethylene glycol, vaseline, paraffin, stearyl alcohol, stearic acid, human serum albumin, mannitol, sorbitol, lactose, sucralose, stevia, aspartame, acesulfame potassium, citric acid, lactic acid, malic acid, tartaric acid, phosphoric acid, acetic acid, fruit juice, vegetable juice, etc.

### (Dosage form or shape)

The food composition of the present invention may be prepared in an arbitrary form such as solid, liquid, mixture, suspension, powder, granule, paste, jelly, gel, and capsule. The food composition of the present invention can be made in such an arbitrary form as dairy product, supplement, confectionery, drink, drinkable preparation, seasoning, processed food, daily dish, and soup. More specifically, the composition of the present invention may be in the form of liquid diet, semi-liquid diet, jelly, gel, powder, special formula powdered milk, special formula liquid milk, powdered and liquid milk for pregnant and nursing women, fermented milk, bar, mousse, chocolate, biscuit, ice cream, fermented milk, lactic acid bacteria beverage, lactic beverage, milk beverage, soft drink, fruit juice beverage, tablet, cheese, bread, biscuit, cracker, pizza crust, food for sick persons, nutritional food, frozen food, processed food, or the like, or may be in a form of granule, powder, paste, thick solution or the like for being mixed in drink or food and then administered. Granules and powders can be in the form of cubes or sticks (single-dose packets). For the present invention, special formula powdered milk means a product obtained by processing raw milk, cow's milk, special cow's milk, or raw water buffalo's milk, or a food made from any of such milks as a raw material or using such a food as a main raw material, adding nutrients necessary for infants and young children to the processed product, and making the resulting composition into powder as defined in the "Ministerial Ordinance on Milk and Milk products Concerning Compositional Standards, etc. (hereinafter referred to as the "Ministerial Ordinance on Milk, etc.")". For the present invention, special formula liquid milk means a product obtained by processing raw milk, cow's milk, special cow's milk, or raw water buffalo's milk, or a food made from any of such milks as a raw material or using such a food as a main raw material, adding nutrients necessary for infants and young children to the processed product, and making the resulting composition into liquid as defined in the Ministerial Ordinance on Milk, etc.

The pharmaceutical composition of the present invention can be in an arbitrary dosage form, for example, those suitable for oral administration including solid preparations such as tablet, granule, powder, pill, and capsule and liquid preparations such as solution, suspension, and syrup, gel, aerosol, or the like.

### (Others)

In the production of the composition of the present invention, time of adding the active ingredient can be appropriately chosen. Unless the characteristics of the active ingredient are not markedly degraded, the time of the addition is not particularly limited. For example, the active ingredient can be blended by mixing it with raw materials. Alternatively, the active ingredient can be added at the final stage of the production to produce a composition comprising the active ingredient.

The composition of the invention can have an indication describing the purpose of use (intended use), or an indication describing that administration of the composition is recommended for specific subjects.

The composition of the present invention can have an indication describing that, with the composition or the active ingredient, *Parabacteroides* bacteria in the intestine (intestinal tract or gastrointestinal tract) can be (appropriately) increased, ratio of *Parabacteroides* bacteria in the intestinal flora can be increased, growth of *Parabacteroides* bacteria in the intestine can be promoted, a disease or condition that is ameliorated by promoting growth of *Parabacteroides* bacteria in the intestine can be treated, *Bifidobacterium* bacteria (bifidobacteria) in the intestine (intestinal tract or gastrointestinal tract) can be (appropriately) increased, ratio of *Bifidobacterium* bacteria in the intestinal flora can be increased, growth of *Bifidobacterium* bacteria in the intestine can be promoted, a disease or condition that is ameliorated by promoting growth of *Bifidobacterium* bacteria in the intestine can be treated, the composition can be used as prebiotics, the composition can be used as synbiotics, inflammation, chronic obstructive pulmonary disease (COPD), obesity, abnormal glucose metabolism, multiple sclerosis, hepatic steatosis, and cancer can be treated, a favorable intestinal environment can be maintained, intestinal flora can be regulated, stomach and bowels can be regulated, bowel movements can be improved, or intestinal environment can be improved, and the composition of the present invention can also have an indication describing that administration of the composition is recommended to specific subjects. The indication may be a direct or indirect indication. Examples of the direct indication include descriptions on tangible articles such as the product itself, package, container, label, and tag, and examples of the indirect indication includes advertising and campaign activities using such places or means as web site, shop, pamphlet, exhibition, seminar such as media seminar, book, newspaper, magazine, television, radio, postal matter, E-mail, and sound.

In one embodiment, the indication of the recommendation to consume the composition is individually. Such an indication may be made in writing (whether written or electronic) addressed to a subject, terminal of a subject, such as tablet, smart phone, and personal computer, or by using a social networking site of a subject, etc. Such an indication can also be made together with an indication of the results of any tests and analyses, such as intestinal flora test, fecal material test, and fecal metabolome analysis for a subject.

### [Other aspects]

As another aspect, the present invention provides a food information-providing apparatus comprising the followings:
an information acquisition part that acquires information on intestinal flora of a subject;
a derivation part that derives information on a food to be suggested to the subject on the basis of the information on the intestinal flora; and
a provision part that provides the derived food information on the food to the subject.

In a preferred embodiment, the acquisition part of the apparatus acquires information on the presence or absence or amount of *Parabacteroides* bacteria from the information on the intestinal flora, and the derivation part derives information on the food that is a composition comprising any selected from the group consisting of kojibiose and oligosaccharides having kojibiose as a constituent sugar on the basis of the information on the presence or absence or amount of *Parabacteroides* bacteria. Alternatively, the acquisition part of the apparatus acquires information on the presence or absence or amount of *Bifidobacterium* bacteria from the information on the intestinal flora, and the derivation part derives information on the food that is a composition comprising kojibiose on the basis of the information on the presence or absence or amount of *Bifidobacterium* bacteria.

In one embodiment, the information on the food to be provided can be displayed on a terminal of a subject. In another embodiment, the apparatus further comprises a display part that displays the information on the food to be provided. The display part can be a terminal of a subject, such as a tablet, smart phone, and personal computer.

In one embodiment, the food information-providing apparatus may have an analysis part that analyzes the bacterial flora for a sample obtained from a subject, and may also have an order-receiving part that receives orders for foods from a subject on the basis of information about the proposed foods.

As another aspect, the present invention provides a method for proposing a food, which comprises the following steps of:
acquiring information on intestinal flora of a subject;
deriving information on a food to be suggested to the subject on the basis of the information on the intestinal flora; and
providing the derived information on the food to the subject.

In a preferred embodiment, in the step of acquiring the information, information on the presence or absence or amount of *Parabacteroides* bacteria is acquired from the information on the intestinal flora, and in the step of deriving information on the food, information on the food that is a composition comprising any selected from the group consisting of kojibiose and oligosaccharides having kojibiose as a constituent sugar is derived on the basis of the information on the presence or absence or amount of *Parabacteroides* bacteria. Alternatively, in the step of acquiring the information, information on the presence or absence or amount of *Bifidobacterium* bacteria is acquired from the information on the intestinal flora, and in the step of deriving information on the food, information on the food that is a composition comprising galactosylkojibiose is derived on the basis of the information on the presence or absence or amount of *Bifidobacterium* bacteria.

Such a method as described above may further comprise the step of displaying the information on the food to be provided on a terminal of a subject. The terminal of a subject may be, for example, a tablet, smart phone, or personal computer.

Hereafter, the present invention will be more specifically explained with reference to the following examples. However, the technical scope of the present invention is not limited by these examples.

### Examples

### [Test Example 1]

### (Fecal culture)

Each substance to be evaluated was added at a concentration of 1% to a diluted fecal solution in which feces of healthy adult origin were suspended in the GAM Semisolid without Dextrose "Nissui" (Nissui Pharmaceutical Co., Ltd.) for GAM glycolysis from which agar was removed by filtration.

The following substances were used as the substances to be evaluated.
Oligosaccharide composition
Galactosylkojibiose (trisaccharide fraction)
Tetrasaccharide fraction
Kojibiose
Fructooligosaccharides (comparative example)
Isomaltose (comparative example)

The oligosaccharide composition was the enzymatic reaction solution prepared in Production Example 1 described below (containing 30 to 35% of 4'-galactosylkojibiose (α-D-Glcp-(1→2)-[β-D-Galp-(1→4)-]D-Glcp), 30 to 35% of lactose, and 25 to 30% of fructose based on solid content). The galactosylkojibiose (trisaccharide fraction) refers to the fractionated and purified product containing 4'-galactosylkojibiose as the main ingredient prepared in Production Example 2. The tetrasaccharide fraction refers to the fractionated and purified product containing a tetrasaccharide consisting of 4'-galactosylkojibiose attached by one molecule of glucose as the main ingredient prepared in Production Example 2.

As kojibiose, fructooligosaccharides, and isomaltose, the followings were used, respectively.
Kojibiose: Kojibiose produced by Fujifilm Wako Pure Chemicals Corporation, distributor code 116-00953
Fructooligosaccharides (comparative example): Fructooligosaccharides produced by Meiji Co., Ltd., containing 85% or more of GF2 (kestose), the remainder consists of GF3, etc.
Isomaltose (comparative example): "Isomaltose" produced by Hayashibara Corporation, code IM121

Fecal materials of two individuals were each tested without mixing them. The medium used for the test was allowed to stand in an anaerobic glove box at least overnight before use. For the culture, a microbioreactor BioLector Pro (m2p-Labs) and a plate with microfluidic channels were used, and the culture was performed for 2 days at 37°C and 600 rpm in an anaerobic environment while maintaining a constant pH of 6.5 with 1.5 N HCl and sodium hydroxide during the culture. The prebiotic abilities of the added substances were evaluated by comparing the bacterial flora obtained after the culture with that obtained under the control condition in which the substances to be evaluated were not added.

### (Relative abundance analysis of intestinal bacteria with next generation sequencer)

DNA was extracted and purified from the diluted fecal solutions after the culture by using Maxwell RSC PureFood GMO & Authentication Kit (Promega), and amplicon sequencing of the V3-V4 region was performed by using MiSeq (Illumina). The resulting fastq files were analyzed with QIIME2 (https://qiime2.org/) to calculate the relative abundance of each intestinal bacterium.

The results are shown in Figs. 1-1 and 1-2.

For the *Parabacteroides* bacteria, an increase in relative abundance was observed in all of the intestinal floras of the two subjects to which the oligosaccharide composition, kojibiose, galactosylkojibiose (trisaccharide fraction), and tetrasaccharide fraction were added, respectively.

The *Parabacteroides* bacteria are bacteria that inhabit in the intestines. Promotion of growth of *Parabacteroides* bacteria is expected to have effects against inflammation, obesity, abnormal glucose metabolism, hepatic steatosis, and cancer (Non-patent documents 1 to 3). In addition, it has been reported that *P. distasonis* is decreased in patients with multiple sclerosis (MS) (Non-patent document 4), and patients with non-alcoholic fatty liver disease (NAFLD) and healthy controls (CTRLs) can be distinguished on the basis of the combination of bacteria of the genus *Parabacteroides* with bacteria of the genera *Oscillospira, Rickenellaceae, Parabacteroides,* etc. (Non-patent document 5). Therefore, from the results shown in Fig. 1-1, it is considered that the oligosaccharide composition, kojibiose, galactosylkojibiose (trisaccharide fraction), and tetrasaccharide fraction can be used for treating any selected from the group consisting of inflammation, obesity, abnormal glucose metabolism, multiple sclerosis (MS), hepatic steatosis, and cancer.

It has also been reported that oral administration of *Parabacteroides goldsteinii* ameliorated weight loss and elevated inflammatory cytokines in lung tissue in a mouse model of smoking-induced chronic obstructive pulmonary disease (COPD), and it has been suggested that, as the mechanism of the above amelioration, pentaacyl-type lipopolysaccharides, cell wall components of *Parabacteroides goldsteinii,* antagonize the activation of TLR4 receptors by inflammatory hexaacyl-type lipopolysaccharides derived from intestinal bacteria (Non-patent document 6). Therefore, from the results shown in Fig. 1-1, it is considered that the oligosaccharide composition, kojibiose, galactosylkojibiose (trisaccharide fraction), and tetrasaccharide fraction can be used for treating chronic obstructive pulmonary disease (COPD).

For the *Bifidobacterium* bacteria, an increased relative abundance was observed in all of the intestinal floras of the two subjects to which the oligosaccharide composition, kojibiose, galactosylkojibiose (trisaccharide fraction), and tetrasaccharide fraction were added, respectively. For all of samples of the two subjects, galactosylkojibiose and oligosaccharide composition more significantly increased the relative abundance of *Bifidobacterium* bacteria compared with kojibiose.

It is known that bifidobacteria themselves or the short-chain fatty acids produced by bifidobacteria produce an intestine-regulating effect, and that the production of short-chain fatty acids lowers the pH in the colon, resulting in increased solubility of minerals, which facilitates their passage through mucosal cells and promotes their absorption into the cells (Non-patent document 8). Therefore, from the results shown in Fig. 1-2, the oligosaccharide composition, kojibiose, galactosylkojibiose (trisaccharide fraction), and tetrasaccharide fractions can be used for intestinal regulation and to promote mineral absorption. Examples of such minerals include calcium, magnesium, iron, potassium, zinc, copper, chromium, manganese, molybdenum, iodine, sodium, phosphorus, and selenium. The composition can be expected to promote the absorption of, in particular, calcium, magnesium, and iron, among them.

### [Test Example 2: Human study (changes in intestinal flora after 2 weeks of ingestion of oligosaccharide composition)]

Changes in intestinal flora of adult males were evaluated after they were fed 20 g per day of a sugar solution containing galactosylkojibiose shown in Production Example 1 described below for 2 weeks. DNA extraction and purification from diluted fecal solutions, amplicon sequencing, and data analysis were performed in the same manners as in Test Example 1.

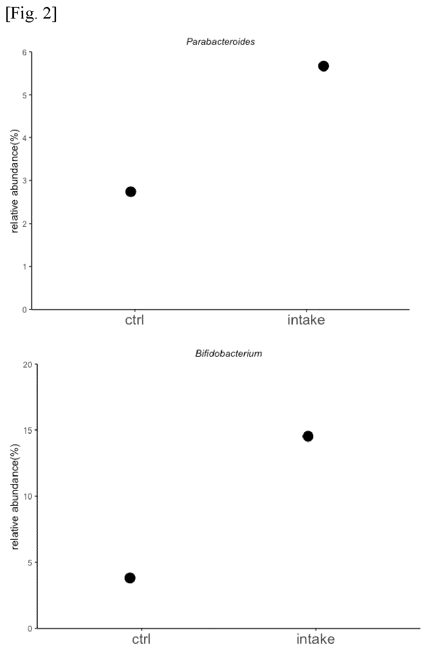

The results are shown in Fig. 2. Increases in relative abundances of *Parabacteroides* and *Bifidobacterium* bacteria were observed.

### [Test Example 3]

### (Fecal culture)

Galactosylkojibiose (trisaccharide fraction) was added at a concentration of 0.25% to a diluted fecal solution in which feces of healthy adult origin were suspended in the GAM Semisolid without Dextrose "Nissui" (Nissui Pharmaceutical Co., Ltd.) from which agar was removed by filtration (n=60). The galactosylkojibiose (trisaccharide fraction) was the fractionated and purified product containing 4'-galactosylkojibiose as the main ingredient prepared in Production Example 2.

The fecal samples for 60 subjects were aliquoted under refrigerated conditions immediately after excretion and frozen at -80°C until use. The test was performed for each of the fecal samples without mixing them. The medium used for the test was allowed to stand in an anaerobic glove box at least overnight before use. For the culture, a 96-well deepwell plate was used, and the culture was performed for 1 day at 37°C in an anaerobic environment. The prebiotic ability of the added evaluation substance was evaluated by comparing the bacterial flora observed after the culture with that obtained under the control condition in which distilled water was added instead of the evaluation substance.

### (Relative abundance analysis of intestinal bacteria with next generation sequencer)

DNA was extracted and purified from the diluted fecal solutions after the culture by using Maxwell RSC PureFood GMO & Authentication Kit (Promega), and amplicon sequencing of the V3-V4 region was performed by using MiSeq (Illumina). The resulting fastq files were analyzed with QIIME2 (https://qiime2.org/) to calculate the relative abundance of each intestinal bacterium.

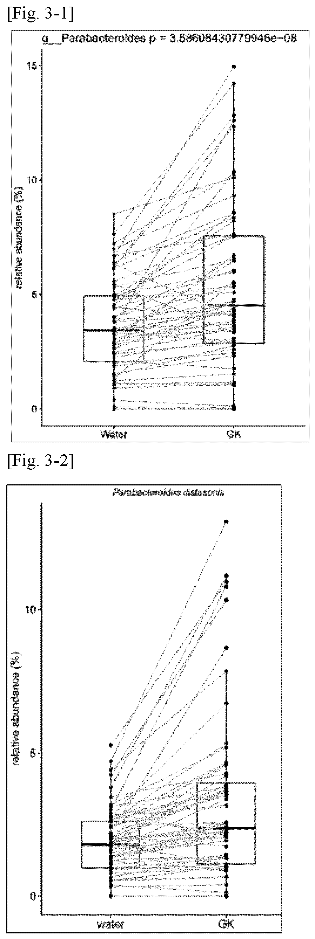

The output results at the genus level are shown in Fig. 3-1. As in Test Example 1, a significant increase in the relative abundance of *Parabacteroides* bacteria was observed with the addition of galactosylkojibiose (trisaccharide fraction) (Wilcoxon's signed rank test, p = 3.59 x 10⁻⁸).

Species-level analysis by amplicon sequencing of the V3-V4 region with MiSeq (Illumina) showed a significant increase in the species indicated as *"Parabacteroides_"* (Wilcoxon's signed rank order test, p = 2.07 x 10⁻⁸). The results are shown in Fig. 3-2.

*Parabacteroides distasonis* is one of the two major species in human feces, and amplicon sequencing of the V3-V4 region was performed with MiSeq (Illumina) for DNA extracted from a reference strain of *Parabacteroides distasonis* (JCM 5825T) in the same manner as used for DNA obtained from the diluted fecal solution after the culture. The resulting fastq files were analyzed with QIIME2 (https://qiime2.org/), and confirmed that it was classified as that of *"Parabacteroides_"* at the bacterial species level. For the other major species, *Parabacteroides merdae,* DNA extracted from the reference strain (JCM 9497T) thereof was not analyzed as that of *"Parabacteroides_",* but analyzed as that of *"Parabacteroides merdae".*

In addition, when DNAs extracted from fecal samples provided from 50 volunteers were analyzed for flora by both amplicon sequencing of the V3-V4 region using MiSeq (Illumina) and shotgun metagenomics in the past, it was confirmed that the species indicated as *"Parabacteroides_"* strongly corresponded to *Parabacteroides distasonis* obtained by the shotgun metagenomics.

On the basis of the above results, it was judged that the results shown in Fig. 3-2 indicate that the addition of galactosylkojibiose (trisaccharide fraction) significantly increased the relative abundance of *Parabacteroides distasonis* in the feces, and the species is indicated as *Parabacteroides distasonis* also in the diagram.

### (Subsummary)

Also in Test Example 3, increase in the relative abundance of *Parabacteroides* bacteria was observed in the feces cultured with the addition of galactosylkojibiose (trisaccharide fraction), confirming the growth-promoting effect thereof for the *Parabacteroides* bacteria. It was also confirmed that the addition of galactosylkojibiose (trisaccharide fraction) significantly increased the relative abundance of at least *Parabacteroides distasonis* among the *Parabacteroides* bacteria.

### [Production Example 1: Production of oligosaccharide composition]

### (Preparation of enzyme solution)

*Lactobacillus satsumensis* JCM12392 activation-cultured in a commercial MRS liquid medium was inoculated to the following medium in an amount of 1%, and cultured at 30°C for 27 hours in a jar fermenter while maintaining a constant pH of 6.4 with sodium hydroxide, and then the culture system was cooled to 10°C or lower to terminate the culture.

**[Table 1]**

| Medium composition | % |
|---|---|
| Glucose | 6.5 |
| Yeast peptone HYP-A581 (Oriental Yeast) | 0.75 |
| Yeast extract Gistex LS powder (DSM) | 1.5 |
| East Rich Manganese (Oriental Yeast) | 0.2 |
| POLYALDO 10-1-0 (Lonza) | 0.1 |

The resulting culture medium was centrifuged, and the supernatant was discarded, thereby concentrating the bacterial cell density 15-fold to make an enzyme solution.

### (Enzymatic reaction)

Sucrose, lactose, and the enzyme solution were mixed with mineral water at concentrations in the mixture of 30 w/w %, 30 w/w %, and 0.5 w/w %, respectively, and the reaction was allowed at 48°C for 3 days under static conditions to obtain an enzymatic reaction solution (oligosaccharide composition, comprising 30 to 35% of 4'-galactosylkojibiose, 30 to 35% of lactose, and 25 to 30% of fructose based on solid content).

### [Production Example 2: Production of purified trisaccharide and tetrasaccharide fractions]

### (Preparation of enzyme solution)

*Lactobacillus satsumensis* JCM12392 was cultured overnight at 30°C in the commercial MRS liquid medium, and then the culture system was cooled below 10°C to terminate the culture.

The resulting culture medium was centrifuged, the supernatant was discarded to collect the bacterial cells, then an equal volume of a citrate-phosphate buffer (pH 5.0) was added, and the bacterial cells were collected again to wash them. The above buffer in the half volume of the original culture medium was added to the bacterial cells, and the suspension was used as the enzyme solution.

### (Enzymatic reaction)

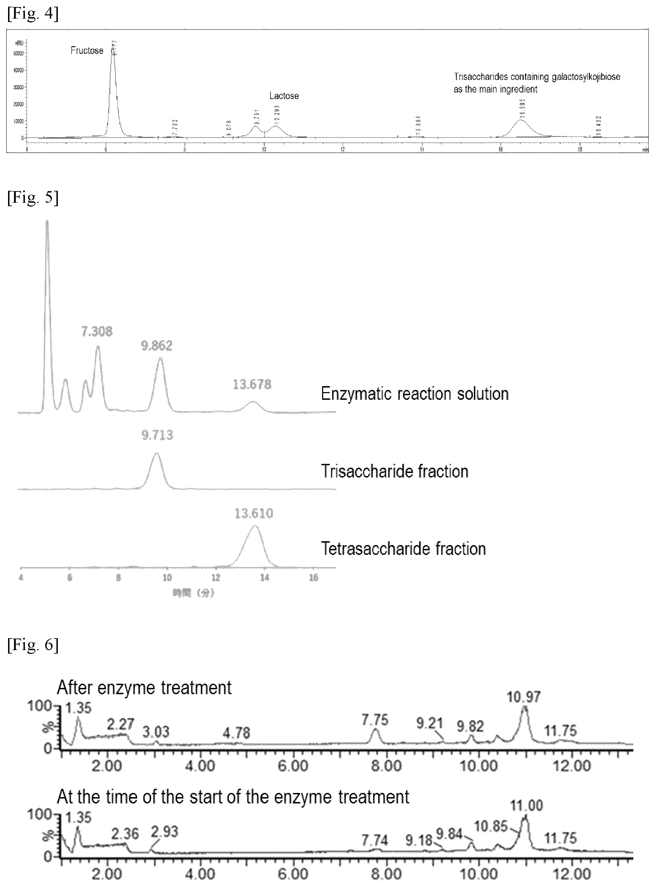

Sucrose, lactose, and the enzyme solution were mixed with mineral water at concentrations in the mixture of 32 w/w %, 16 w/w %, and 10 w/w %, respectively, and the enzymatic reaction was allowed at 48°C for 18 hours under static conditions. The sugar composition of the reaction solution after the reaction was analyzed by using HPLC (SUPELCO apHera (registered trademark) NH₂ HPLC Column, mobile phase 68% acetonitrile, column temperature 35°C, flow rate 1.0 mL/minute, RI detection), and the results are shown in Fig. 4. The composition contained 25 to 30% of 4'-galactosylkojibiose, 15 to 20% of lactose, and 30 to 35% fructose based on solid content.

### (Purification of trisaccharide and tetrasaccharide fractions)

The enzymatic reaction solution was passed through a carbon-celite column, and then non-adsorbed substances were removed by passing distilled water through the column. By eluting stepwise with ethanol solutions of varying concentrations, a trisaccharide fraction containing 4'-galactosylkojibiose as the main ingredient and a tetrasaccharide fraction containing a tetrasaccharide consisting of 4'-galactosylkojibiose attached by one molecule of glucose were obtained (Fig. 5).

### (Lactase treatment and structural analysis of fractionation and purification products)

The purified trisaccharide fraction was lyophilized and dissolved in a phosphate-citrate buffer, pH 6.5 at a concentration of 10%. Then, 0.1% of lactase (product name GODO-YNL, Godo Shusei Co., Ltd.) was added to the solution, and the enzymatic reaction was allowed at 40°C. LCMS analysis using an amide column (BEH Amide, Waters) showed the formation of kojibiose (disaccharide consisting of glucoses linked by α-1,2 linkage, corresponding to the elution time of 7.75 minutes) after the reaction (Fig. 6).

On the basis of the above results and others, it was confirmed that the main ingredient of the trisaccharide fraction was 4'-galactosylkojibiose. From the activity of the enzyme, transfer of lactose to galactose residues is also possible, but surprisingly, transfer to galactose residues scarcely occurred, and galactosylkojibiose was formed (see below).

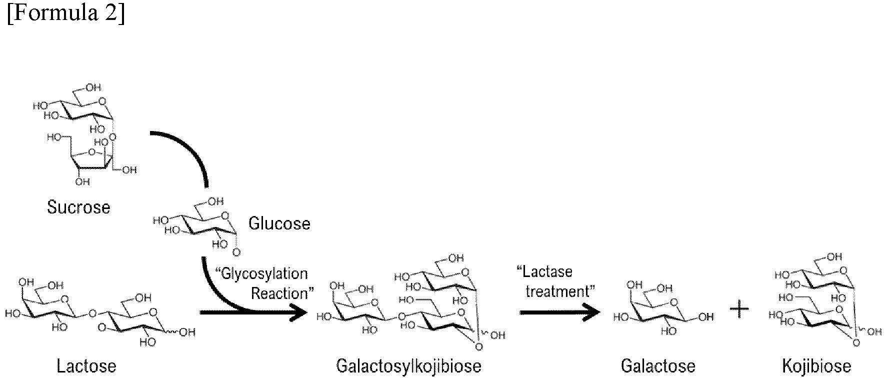

### [Production Example 3: 100% Orange juice drink]

1/6 Concentrated orange juice (168 g), the oligosaccharide composition obtained in Production Example 1 (20 g, solid content 60%), and an appropriate amount of flavoring are dissolved in ion-exchanged water to make a total volume of 1000 ml. This solution is filled in a container and heat-sterilized at 65°C for 10 minutes to obtain a 100% orange juice drink containing the oligosaccharide composition obtained in Production Example 1. This fruit juice drink contains about 0.36 to 0.42 g/100 g of 4'-galactosylkojibiose. This fruit juice drink can be used to improve the intestinal flora.

### [Example 4: Yogurt drink]

Raw material cream, nonfat concentrated milk, and raw material water are mixed so that soluble matters are dissolved, heat sterilized at 110°C for 30 seconds, and cooled to 43°C, then lactic acid bacteria starter consisting of *Lactobacillus bulgaricus* and *Streptococcus thermophilus* is added to the mixture, and fermentation is allowed to an acidity of 0.75 to homogenize the mixture. The homogenized mixture is mixed with raw material water, pectin, sucralose, the oligosaccharide composition obtained in Production Example 1, and flavoring so that soluble matters are dissolved, and heat-sterilized at 80°C for 10 minutes, then the mixture is stirred under cooling and used. The mixing ratios of the raw materials are shown in the following table. The resulting yogurt drink can be used to promote growth of *Bifidobacterium* bacteria in the intestines.

**[Table 2]**

| Raw material | Mixing ratio (%) |
|---|---|
| Raw material cream | 1.008 |
| skim milk powder | 22.01 |
| Lactic acid bacteria starter | 1.2 |
| Oligosaccharide composition | 5.5 |
| Sucralose | 0.0064 |
| Pectin | 0.25 |
| Flavoring | 0.03 |
| Raw material water | 70.0 |
| Total | 100 |

### [Example 5: Fermented milk 1]

Raw milk (500.0 g), skimmilk powder (53.2 g), fresh cream (23.0 g), tap water (403.6 g), and sucrose (50 g) are mixed to prepare raw material milk. The raw material milk is heat-sterilized at 95°C, and the heat-sterilized raw material milk is cooled. Then, 0.5% of *Liquorilactobacillus satsumensis* concentrate is inoculated to the raw material milk, fermentation is allowed ed at 48°C for 18 hours under static conditions, and *Lactobacillus bulgaricus* and *Streptococcus thermophilus* are added as lactic acid bacteria starter. The addition amount of the lactic acid bacteria starter is 20 g. The raw material milk to which the lactic acid bacteria starter has been added is filled into a cup container (volume 100 ml, made of plastic). The raw material milk filled into the cup container is fermented under static conditions in a fermentation chamber at 43°C until the lactic acidity reaches 0.7%. The resulting fermented milk can be used to promote mineral absorption.

### [Example 6: Fermented milk 2]

Raw milk (500.0 g), skimmilk powder (53.2 g), fresh cream (23.0 g), tap water (403.6 g), and the oligosaccharide composition obtained in Production Example 1 (54 g) are mixed to prepare raw material milk. Then, the raw material milk is heat-sterilized at 95°C, and the heat-sterilized raw material milk is cooled to a temperature of 43°C. Then, *Lactobacillus bulgaricus* and *Streptococcus thermophilus* are added to the heat-sterilized raw material milk as lactic acid bacteria starter. The addition amount of the lactic acid bacteria starter is 20 g. The raw material milk to which the lactic acid bacteria starter has been added is filled into a cup container (volume 100 ml, made of plastic). The raw material milk filled into the cup container is fermented under static conditions in a fermentation chamber at 43°C until the lactic acidity reaches 0.7%. The resulting fermented milk can be used to regulate intestines.

### Industrial Applicability

According to the present invention, maintenance and improvement of people's health is supported by a composition for improvement of intestinal flora, containing any selected from the group consisting of kojibiose and oligosaccharides having kojibiose as a constituent sugar. According to the present invention, a food composition that supports maintenance and improvement of people's health and a method for producing such a food can also be provided. Furthermore, according to the present invention, for various people, improvement of nutrition can be realized, a healthy life is ensured, and welfare is promoted.

## Claims

1. A composition for improvement of intestinal flora, the composition comprising any selected from the group consisting of kojibiose and oligosaccharides having kojibiose as a constituent sugar.

2. The composition according to claim 1, wherein the improvement of intestinal flora includes promoting growth of *Parabacteroides* bacteria.

3. A composition for promoting growth of *Bifidobacterium* bacteria in the intestine, the composition comprising galactosylkojibiose.

4. The composition according to any one of claims 1 to 3, which is for use as prebiotics or synbiotics.

5. A composition for treating any selected from the group consisting of inflammation, chronic obstructive pulmonary disease, obesity, abnormal glucose metabolism, multiple sclerosis, hepatic steatosis, and cancer, the composition comprising any selected from the group consisting of kojibiose and oligosaccharides having kojibiose as a constituent sugar.

6. The composition according to claim 5, wherein the treatment is carried out through promoting growth of *Parabacteroides* bacteria in the intestine.

7. A composition for treating a disease or condition that is ameliorated by promoting growth of *Parabacteroides* bacteria in the intestines, the composition comprising any selected from the group consisting of kojibiose and oligosaccharides having kojibiose as a constituent sugar.

8. A composition for promoting mineral absorption or intestinal regulation, the composition comprising galactosylkojibiose.

9. The composition according to claim 8, wherein the mineral absorption promotion or intestinal regulation is carried out through promoting growth of *Bifidobacterium* bacteria.

10. A composition for treating a disease or condition that is ameliorated by promoting growth of *Bifidobacterium* bacteria in the intestines, the composition comprising galactosylkojibiose.

11. The composition according to any one of claims 1 to 3 and 5 to 10, which comprises any selected from the group consisting of kojibiose and oligosaccharides having kojibiose as a constituent sugar as a composition comprising galactosylkojibiose obtained by allowing lactic acid bacteria that express a glucansucrase or a processed product thereof to act on a composition comprising sucrose and lactose.

12. The composition according to claim 4, which comprises any selected from the group consisting of kojibiose and oligosaccharides having kojibiose as a constituent sugar as a composition comprising galactosylkojibiose obtained by allowing lactic acid bacteria that express a glucansucrase or a processed product thereof to act on a composition comprising sucrose and lactose.

13. The composition according to any one of claims 2, 6 and 7, wherein the *Parabacteroides* bacteria contain *Parabacteroides distasonis.*

14. A food information-providing apparatus comprising:
an information acquisition part that acquires information on intestinal flora of a subject;
a derivation part that derives information on a food to be suggested to the subject on the basis of the information on the intestinal flora; and
a provision part that provides the derived information on the food to the subject, wherein
the information acquisition part acquires information on the presence or absence or amount of *Parabacteroides* bacteria from the information on the intestinal flora, and the derivation part derives information on the food that is a composition comprising any selected from the group consisting of kojibiose and oligosaccharides having kojibiose as a constituent sugar on the basis of the information on the presence or absence or amount of *Parabacteroides* bacteria, or
the information acquisition part acquires information on the presence or absence or amount of *Bifidobacterium* bacteria from the information on the intestinal flora, and the derivation part derives information on the food that is a composition comprising galactosylkojibiose on the basis of the information on the presence or absence or amount of *Bifidobacterium* bacteria.

15. The apparatus according to claim 14, wherein the information on the food to be provided can be displayed on a terminal of the subject.

16. The apparatus according to claim 14, which further comprises a display part that displays the information on the food to be provided.

17. A method for providing information on a food, the method comprising
acquiring information on intestinal flora of a subject;
deriving information on a food to be suggested to the subject on the basis of the information on the intestinal flora; and
providing the derived information on the food to the subject,
wherein
in the step of acquiring the information, information on the presence or absence or amount of *Parabacteroides* bacteria is acquired from the information on the intestinal flora, and in the step of deriving the information on the food, information on the food that is a composition comprising any selected from the group consisting of kojibiose and oligosaccharides having kojibiose as a constituent sugar is derived on the basis of the information on the presence or absence or amount of *Parabacteroides* bacteria, or
in the step of acquiring the information, information on the presence or absence or amount of *Bifidobacterium* bacteria is acquired from the information on the intestinal flora, and in the step of deriving the information on the food, information on the food that is a composition comprising galactosylkojibiose is derived on the basis of the information on the presence or absence or amount of *Bifidobacterium* bacteria.

18. The method according to claim 17, which further comprises the step of displaying the information on the food to be provided on a terminal of the subject.
